(19) Europäisches Patentamt
European Patent Office
Office européen des brevets

(11) **EP 1 036 785 B1**

(12) **EUROPÄISCHE PATENTSCHRIFT**

(45) Veröffentlichungstag und Bekanntmachung des Hinweises auf die Patenterteilung:
**10.09.2003 Patentblatt 2003/37**

(51) Int Cl.[7]: **C07C 209/48**, C07C 211/27

(21) Anmeldenummer: **00103365.3**

(22) Anmeldetag: **22.02.2000**

(54) **Verfahren zur Herstellung von Bistrifluormethylbenzylaminen**

Process for the preparation of bis(trifluoromethyl)benzylamines

Procédé pour la préparation de bis(trifluorométhyl)benzylamines

(84) Benannte Vertragsstaaten:
**CH DE FR GB IT LI**

(30) Priorität: **02.03.1999 DE 19908943**

(43) Veröffentlichungstag der Anmeldung:
**20.09.2000 Patentblatt 2000/38**

(73) Patentinhaber: **Bayer Aktiengesellschaft**
**51368 Leverkusen (DE)**

(72) Erfinder:
- **Marhold, Albrecht, Dr.**
  **51373 Leverkusen (DE)**
- **Kysela, Ernst, Dr.**
  **51427 Bergisch Gladbach (DE)**

(56) Entgegenhaltungen:
**US-A- 3 726 969**

- **DATABASE CHEMABS [Online] CHEMICAL ABSTRACTS SERVICE, COLUMBUS, OHIO, US UEHARA, RYOICHI ET AL: "Cobalt catalyst" retrieved from STN Database accession no. 92:100139 XP002133412 & JP 54 037593 B (NIKKO SCIENTIFIC AND CHEMICAL INDUSTRIES, LTD., JAPAN) 15. November 1979 (1979-11-15)**
- **DATABASE WPI Section Ch, Week 198729 Derwent Publications Ltd., London, GB; Class B05, AN 1987-202004 XP002133413 & JP 62 129257 A (DAICEL CHEM IND LTD), 11. Juni 1987 (1987-06-11)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 036 785 B1

**Beschreibung**

Die vorliegende Erfindung betrifft ein verbessertes Verfahren zur Herstellung von Bistrifluormethylbenzylaminen durch Hydrierung der entsprechenden Bistrifluormethylbenzonitrile.

Bistrifluormethylbenzylamine und insbesondere 3,5-Bistrifluormethylbenzylamin haben Bedeutung als Pflanzenschutz- und pharmazeutische Wirkstoffe und als Vorprodukte dafür.

In älterer Literatur (siehe J. A.C. S. 66, 725 (1944) und 82, 2386 (1960)) sowie Chem. Ber. 85, 325 (1952) ist beschrieben, dass man aliphatische Nitrile und Indolylacetonitrile durch katalytische Reduktion mit Wasserstoff in Gegenwart von Raney-Nickel oder Rhodium in die entsprechenden Amine überführen kann. Soweit überhaupt Lösungsmittel zum Einsatz kommen, sind dies Alkohole. Gegebenenfalls kann in Gegenwart von Ammoniak gearbeitet werden.

Für die Herstellung von Trifluormethylbenzylaminen wird die katalytische Reduktion von Trifluormethylbenzonitrilen in Gegenwart von Rhodium- oder Palladium-Katalysatoren, Ethanol und Chlorwasserstoff beschrieben, wobei die Amine als Hydrochloride anfallen. Diese Hydrochloride müssen auf umständliche Weise gereinigt und anschließend in die freien Amine überführt werden (siehe J. Pharm. Sci. 54, 1204 (1965)).

Gemäß der US-PS 3 726 969 wird das zuletzt genannte Verfahren auch zur Herstellung von 3,5-Bistrifluormethylbenzylamin verwendet. Auch hier sind umständliche Reinigungsschritte vorzunehmen. Gemäß diesem Verfahren ist nur das Aminhydrochlorid, nicht jedoch das freie Amin hergestellt worden.

JP-A-62 129 257 offenbart die Hydrierung von Benzonitril in Gegenwart von Raney-Kobalt, Ethanol und Ammoniak.

Es besteht deshalb noch das Bedürfnis nach einem Verfahren zur Herstellung von Bistrifluormethylbenzylaminen, bei dem man auf einfache Weise die freien Amine in hohen Reinheiten erhält.

Es wurde nun ein Verfahren zur Herstellung von Bistrifluormethylbenzylaminen durch katalytische Hydrierung der entsprechenden Bistrifluormethylbenzonitrile gefunden, das dadurch gekennzeichnet ist, dass man in Gegenwart von Raney-Kobalt, mindestens einem aliphatischen Ether und Ammoniak arbeitet.

In das erfindungsgemäße Verfahren kann man beispielsweise Bistrifluormethylbenzonitrile der Formel (I) einsetzen

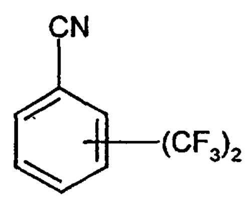

(I)

und Bistrifluormethylbenzylamine der Formel (II) herstellen

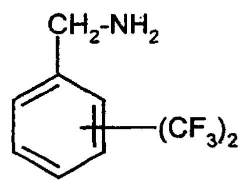

(II).

Vorzugsweise werden Bistrifluormethylbenzonitrile der Formel (III) eingesetzt

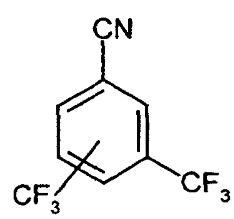

(III)

und Bistrifluormethylbenzylamine der Formel (IV) hergestellt

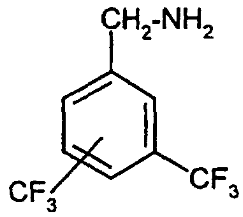

(IV).

Besonders bevorzugt wird 3,5-Bistrifluormethylbenzonitril eingesetzt und 3,5-Bistrifluormethylbenzylamin hergestellt.

Bistrifluormethylbenzonitrile sind entweder kommerziell erhältlich oder auf bekannte Weise oder analog dazu herstellbar.

Raney-Kobalt ist ebenfalls kommerziell erhältlich oder kann auf bekannte Weise aus einer Aluminium-Kobalt-Legierung durch Auslaugung des Aluminiums mit wässrigen Alkalien erhalten werden.

Die aliphatischen Ether können linear, verzweigt oder cyclisch sein und beispielsweise 4 bis 10 C-Atome enthalten, wie Diethyl-, Diisopropyl-, Methyl-tert.butyl-, Dibutyl-, Ethylenglykoldimethyl-, Diethylenglykoldimethyl- und Tetraethylenglykoldimethylethern sowie Tetrahydrofüran, 2-Methyltetrahydrofuran und Dioxan. Es können auch Gemische mehrerer aliphatischer Ether eingesetzt werden.

Pro kg Bistrifluormethylbenzonitril kann man beispielsweise 0,3 bis 2 kg aliphatische Ether und 0,05 bis 1 kg Raney-Kobalt (berechnet als Metall) einsetzen. Vorzugsweise betragen diese Mengen 0,5 bis 1,2 kg aliphatische Ether und 0,07 bis 0,2 kg Raney-Kobalt.

Der Ammoniak sollte weitgehend trocken sein und kann in handelsüblicher Form (Reinheiten 99,5 % oder höher) eingesetzt werden. Man kann ihn dem Reaktionsgefäß in flüssiger Form oder gasförmig zuführen. Pro kg Bistrifluormethylbenzonitril kann man beispielsweise 0,2 bis 4 kg Ammoniak einsetzen. Vorzugsweise beträgt diese Menge 0,3 bis 2,5 kg.

Das erfindungsgemäße Verfahren kann man beispielsweise bei 50 bis 150°C und einem Wasserstoffdruck von 20 bis 200 bar durchführen. Bevorzugt sind Temperaturen von 70 bis 110°C und Drucke von 30 bis 150 bar.

Es ist zweckmäßig, die erfindungsgemäße Hydrierung solange durchzuführen, bis sich alles Bistrifluormethylbenzonitril umgesetzt hat. Man kann dies z.B. mittels GC überprüfen. Im allgemeinen hat man nach 3 bis 15 Stunden einen vollständigen Umsatz des Bistrifluormethylbenzonitrils erreicht.

Bei einer bevorzugten Ausführungsform des erfindungsgemäßen Verfahrens arbeitet man wie folgt: Man legt das Bistrifluormethylbenzonitril, den aliphatischen Ether und das Raney-Kobalt in einem Druckgefäß vor und leitet anschließend ein inertes Gas, z.B. Stickstoff, durch das Druckgefäß, um vorhandenen Sauerstoff zu verdrängen. Dann schließt man das Druckgefäß, fügt zunächst den Ammoniak zu, drückt schließlich Wasserstoff auf und erwärmt auf die vorgesehene Reaktionstemperatur. Es ist auch möglich, während der Hydrierung Bistrifluormethylbenzonitril und Ammoniak nachzudosieren und so die Hydrierung weiterzuführen.

Die Aufarbeitung des nach dem Ende der Reaktion vorliegenden Gemisches kann man auf einfache Weise durchführen, beispielsweise indem man zunächst das Reaktionsgefäß abkühlt, z.B. auf 20 bis 50°C und dann durch Entspannung des Drucks den Ammoniak abdampfen läßt. Den Ammoniak kann man gegebenenfalls wiedergewinnen, z.B. durch Kühlung, und im nächsten Ansatz wiederverwenden. Danach kann man den Katalysator entfernen, z.B. durch Filtration. Auch der Katalysator ist wiederverwendbar. Schließlich kann man das vom Katalysator befreite Gemisch destillieren, wobei zunächst der als Lösungsmittel eingesetzte aliphatische Ether übergeht, der ebenfalls wiederverwendbar ist. Durch Fraktionierung bei vermindertem Druck, z.B. bei 10 bis 100 mbar, kann man abschließend das hergestellte freie Bistrifluormethylbenzylamin in Reinheiten von über 99 % und in Ausbeuten von meist über 80 % isolieren.

Mit dem erfindungsgemäßen Verfahren können Bistrifluormethylbenzylamine auf einfache und effektive Weise direkt in freier Form und in hohen Reinheiten erhalten werden. Die primäre Herstellung von Bistrifluormethylbenzylaminhydrochloriden und umständliche Reinigungsverfahren auf dieser Stufe entfallen.

Es ist ausgesprochen überraschend, dass sich bei der erfindungsgemäßen Arbeitsweise diese Vorteile ergeben. Bisher wurden als Lösungsmittel für derartige Hydrierungen ausschließlich Alkohole eingesetzt und speziell für die Herstellung von Trifluormethylbenzylaminen nur Edelmetallkatalysatoren in Gegenwart von Chlorwasserstoff.

Nachdem bis 1960 für die Hydrierung von aliphatischen Nitrilen auch Raney-Nickel-Katalysatoren in Gegenwart von Ammoniak und Alkoholen als Lösungsmittel in Betracht gezogen wurden, hat dann die Entwicklung der Hydrierung von Trifluormethylbenzylaminen eine andere Richtung genommen, nämlich hin zu Edelmetallkatalysatoren in Gegenwart von Chlorwasserstoff und nach wie vor Alkoholen als Lösungsmittel. Es hat deshalb nicht nahegelegen, für die Verbesserung des bekannten Herstellungsverfahren für Bistrifluormethylbenzylamine den Einsatz von Raney-Kobalt in Gegenwart von Ammoniak und unter Verwendung anderer Lösungsmittel in Betracht zu ziehen.

Neben den Einsatz von Raney-Kobalt-Katalysatoren ist beim erfindungsgemäßen Verfahren auch der Einsatz von aliphatischen Ethern ein wesentliches Merkmal. Aliphatische Ether sind bisher weder für die Hydrierung von aliphatischen Nitrilen, noch für die Hydrierung von Trifluormethylbenzonitrilen in Betracht gezogen worden. Wie aus dem Vergleichsbeispiel 1 hervorgeht ergibt die Kombination Raney-Kobalt-Katalysator, Alkohol als Lösungsmittel in Gegenwart von Ammoniak völlig unbefriedigende Ergebnisse, nämlich ein Reaktionsgemisch, das nur zu rund 30 % das gewünschte Bistrifluormethylbenzylamin enthält, jedoch weit über 50 % unerwünschtes Bistrifluormethylbenzyl-bistrifluromethylbenzamidat. Dieses Vergleichsbeispiel zeigt deutlich den mit der erfindungsgemäßen Verwendung von aliphatischen Ethern als Lösungsmittel verbundenen unerwarteten positiven Effekt.

## Beispiele

### Beispiel 1

In einem Autoklaven wurden 500 g 3,5-Bistrifluormethylbenzonitril, 300 ml flüssiger Ammoniak, 300 ml Tetrahydrofuran und 50 g Raney-Kobalt vorgelegt und dann 10 Stunden bei 90°C 115 bar Wasserstoff aufgedrückt. Danach wurde zunächst auf 22°C abgekühlt und über einen Wäscher entspannt, wobei der Ammoniak abdampfte. Es wurde vom Katalyator abgesaugt, eingeengt und der Rückstand über eine Vigreux-Kolonne im Vakuum fraktioniert. Bei einem Druck von 50 mbar und einer Temperatur von 102 bis 105°C übergehend wurden 356 g (70 % d.Th.) 3,5-Bistrifluormethylbenzylamin mit einer Reinheit von 99,4 % (GC, Flächen-%) erhalten. Als Nachlauf wurden bei 20mbar noch 14 g 90 %-iges Produkt aufgefangen. Es hinterblieb ein Destillationsrückstand von 88 g.

Der Nachlauf wurde zur Destillation der nächsten Ansatzes hinzufügt und so insgesamt 90 % der theoretischen Menge Bistrifluromethylbenzylamin in über 99 %-iger Reinheit erhalten.

### Beispiel 2

Analog Beispiel 1 wurden 100 g 3,5-Bistrifluormethylbenzonitril, 300 ml flüssiger Ammoniak, 100 ml Tetrahydrofuran und 50 g Raney-Kobalt vorgelegt, 5 Stunden bei gleichen Bedingungen Wasserstoff aufgedrückt und wie beschrieben aufgearbeitet. Es wurden 84 g (83 % d.Th.) 3,5- Bistrifluormethylbenzylamin mit einer Reinheit von 99,6 % (GC, Flächen-%) und ein Rückstand von 3 g erhalten.

### Beispiel 3

In einem 120 1-Rührauroklaven wurden 40 kg 3,5-Bistrifluormethylbenzonitril, 20 kg Tetrahydrofuran und 4,0 kg Raney-Kobalt vorgelegt und nach dem Spülen mit Stickstoff 23 kg Ammoniak aufgedrückt. Danach wurden bis auf 30 bar Wasserstoff aufgedrückt, auf 90°C erwärmt und weiterer Wasserstoff bis auf 110 bis 120 bar aufgedrückt. Nach 12 Stunden wurde eine Probe gezogen und mit GC auf vollständigen Umsatz geprüft. Bei nicht vollständigem Umsatz wurde unter den vorstehenden Bedingungen solange weiter hydriert bis der Umsatz vollständig war. Nach vollständigem Umsatz wurde zunächst auf 20°C abgekühlt und über einen Wäscher entspannt, wobei der Ammoniak abdampfte. Dann wurde der Katalysator abfiltriert, der Autoklav und der Filterkuchen mit 10 kg Tetrahydrofuran gespült und das mit der Spülflüssigkeit vereinigte Filtrat am Rotationsverdampfer eingeengt. Die so aufkonzentrierten Rohproduktlösungen aus 3 Ansätzen (Details siehe folgende Tabelle) wurden darauf vereinigt und destilliert.

| Ansatz | Menge (kg) | Gehalt an 3,5-Bistrifluormethylbenzylamin (GC, Flächen-%) | 3,5-Bistrifluormethylbenzylamin (kg) |
|---|---|---|---|
| 3-1 | 59,2 | 60,63 | 35,89 |
| 3-2 | 58,6 | 57,80 | 33,87 |
| 3-3 | 60,0 | 59,85 | 35,91 |

Der Ansatz 3-2 wurde aus 39,5 kg 3,5-Bistrifluormethylbenzonitril erhalten.

Die Destillation erfolgte über eine Kolonne mit 2 m Höhe und 100 mm Duchmesser, die mit 7 x 7 mm Wilson-Spiralen gefüllt war. Bei einem Rücklaufverhältnis von 1:6, einer Kopftemperatur von 90 bis 92°C und einem Druck von 36 mbar. wurden in 5 Fraktionen mit Reinheiten von 99,55 bis 99,75 % (GC, Flächen-%) insgesamt 102 kg 3,5-Bistrifluormethylbenzylamin erhalten, was einer Ausbeute von 84 % der Theorie entspricht.

**Beispiel 4**

Analog Beispiel 1 wurden 100 g 2,4-Bistrifluormethylbenzonitril, 200 ml flüssiger Ammoniak, 150 ml Tetrahydrofuran und 30 g Raney-Kobalt vorgelegt, dann Wasserstoff bis 120 bar bei 100°C aufgedrückt.
Die analoge Aufarbeitung ergab 75 g (74,4 %) 2,4-Bistrifluormethylbenzylamin mit einem Siedepunkt von 78°C/10 mbar, $n_{20}^{D}$: 1,4280.

**Vergleichsbeispiel 1** (Lösungsmittel Alkohol statt Ether)

In einem Autoklaven wurden 500 g 3,5-Bistrifluormethylbenzonitril, 300 ml flüssiger Ammoniak, 300 ml Methanol und 25 g Raney-Kobalt vorgelegt und 12 Stunden bei 90°C und 115 bar mit Wasserstoff hydriert. Vor der Aufarbeitung wurde zunächst abgekühlt und über einen Wäscher entspannt, wobei der Ammoniak abdampfte. Es wurde vom Katalyator abfiltriert und eingeengt. Gemäß GC-MS-Analyse waren im Rohprodukt nur 31 Flächen-% 3,5-Bistrifluormethylbenzylamin, jedoch 56 Flächen-% N-3,5-Bistrifluormethylbenzyl-3,5-bistrifluormethylbenzamidat enthalten. Die Destillation bei 3 mbar bis 170°C Sumpftemperatur lieferte 43 g Destillat und es hinterblieb ein Rückstand von 351 g. Das Destillat enthielt 3,5-Bistrifluormethylbenzylamin in einer Menge, die 8% der Theorie entsprach (berechnet auf 100 %-iges Produkt).

## Patentansprüche

1. Verfahren zur Herstellung von Bistrifluormethylbenzylaminen durch katalytische Hydrierung der entsprechenden Bistrifluormethylbenzonitrile, **dadurch gekennzeichnet, dass** man in Gegenwart von Raney-Kobalt, mindestens einem aliphatischen Ether und Ammoniak arbeitet.

2. Verfahren nach Anspruch 1, **dadurch gekennzeichnet, dass** man Bistrifluormethylbenzonitrile der Formel (I) einsetzt

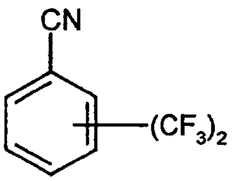

(I)

und Bistrifluormethylbenzylamine der Formel (II) herstellt

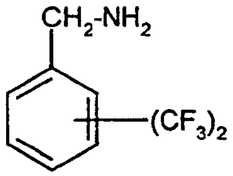

(II).

3. Verfahren nach Ansprüchen 1 und 2, **dadurch gekennzeichnet, dass** man als aliphatische Ether lineare, verzweigte oder cyclische Ether mit 4 bis 10 C-Atomen einsetzt.

4. Verfahren nach Ansprüchen 1 bis 3, **dadurch gekennzeichnet, dass** man 0,3 bis 2 kg aliphatische Ether pro kg Bistrifluormethylbenzonitril einsetzt.

5. Verfahren nach Ansprüchen 1 bis 4, **dadurch gekennzeichnet, dass** man 0,05 bis 1 kg Raney-Kobalt pro kg Bistrifluormethylbenzonitril einsetzt.

6. Verfahren nach Ansprüchen 1 bis 5, **dadurch gekennzeichnet, dass** man 0,2 bis 4 kg Ammoniak pro kg Bistrifluormethylbenzonitril einsetzt.

7. Verfahren nach Ansprüchen 1 bis 6, **dadurch gekennzeichnet, dass** man es bei Temperaturen von 50 bis 150°C und Wasserstoffdrucken von 20 bis 200 bar durchführt.

8. Verfahren nach Ansprüchen 1 bis 7, **dadurch gekennzeichnet, dass** man das nach dem Ende der Reaktion vorliegende Gemisch aufarbeitet, indem man zunächst das Reaktionsgefäß abkühlt, dann durch Entspannung des Druck den Ammoniak verdampfen läßt, danach den Katalysator entfernt und schließlich das vom Katalysator befreite Gemisch destilliert.

## Claims

1. Process for the preparation of bistrifluoromethylbenzylamines by catalytic hydrogenation of the corresponding bistrifluoromethylbenzonitriles, **characterized in that** the process is carried out in the presence of Raney cobalt, at least one aliphatic ether and ammonia.

2. Process according to Claim 1, **characterized in that** the bistrifluoromethylbenzonitriles used are of the formula (I)

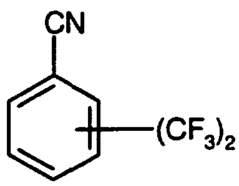

(I)

and the bistrifluoromethylbenzylamines prepared are of the formula (II)

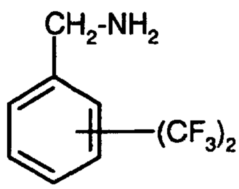

(II).

3. Process according to Claims 1 and 2, **characterized in that** the aliphatic ethers are linear, branched or cyclic ethers having from 4 to 10 carbon atoms.

4. Process according to Claims 1 to 3, **characterized in that** from 0.3 to 2 kg of aliphatic ethers are used per kg of bistrifluoromethylbenzonitrile.

5. Process according to Claims 1 to 4, **characterized in that** from 0.05 to 1 kg of Raney cobalt is used per kg of bistrifluoromethylbenzonitrile.

6. Process according to Claims 1 to 5, **characterized in that** from 0.2 to 4 kg of ammonia are used per kg of bistrifluoromethylbenzonitrile.

7. Process according to Claims 1 to 6, **characterized in that** it is carried out at temperatures of from 50 to 150°C and hydrogen pressures of from 20 to 200 bar.

8. Process according to Claims 1 to 7, **characterized in that** the mixture present at the end of the reaction is worked up by firstly cooling the reaction vessel, then allowing the ammonia to evaporate by releasing the pressure, then removing the catalyst and finally distilling the catalyst-free mixture.

## Revendications

1. Procédé pour la préparation de bistrifluorométhylbenzylamines par hydrogénation catalytique des bistrifluorométhylbenzonitriles

thylbenzonitriles correspondants, **caractérisé en ce que** l'on opère en présence de cobalt de Raney, d'au moins un éther aliphatique et d'ammoniac.

2. Procédé selon la revendication 1, **caractérisé en ce que** l'on part de bistrifluorométhylbenzonitriles de formule (I)

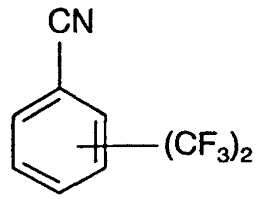

(I)

et on prépare des bistrifluorométhylbenzylamines de formule (II)

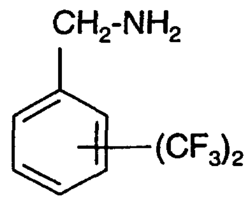

(II)

3. Procédé selon les revendications 1 et 2, **caractérisé en ce que** l'on utilise en tant qu'éthers aliphatiques des éthers linéaires, ramifiés ou cycliques contenant de 4 à 10 atomes de carbone.

4. Procédé selon les revendications 1 à 3, **caractérisé en ce que** l'on utilise de 0,3 à 2 kg d'éther aliphatique par kg de bistrifluorométhyl-benzonitrile.

5. Procédé selon les revendications 1 à 4, **caractérisé en ce que** l'on utilise de 0,05 à 1 kg de cobalt de Raney par kg de bistrifluoro-méthylbenzonitrile.

6. Procédé selon les revendications 1 à 5, **caractérisé en ce que** l'on utilise de 0,2 à 4 kg d'ammoniac par kg de bistrifluorométhyl-benzonitrile.

7. Procédé selon les revendications 1 à 6, **caractérisé en ce que** l'on opère à des températures de 50 à 150°C et des pressions d'hydrogène de 20 à 200 bar.
Le A 33518 (00 103 365.3)

8. Procédé selon les revendications 1 à 7, **caractérisé en ce que**, pour les opérations d'isolement effectuées sur le mélange obtenu après la réaction, on refroidit d'abord le récipient de réaction, on provoque la vaporisation de l'ammoniac par une détente, on élimine ensuite le catalyseur et finalement on distille le mélange débarrassé du catalyseur.